# EUROPEAN PATENT APPLICATION

(11) **EP 4 692 776 A1**
(43) Date of publication of application: **11.02.2026**
(21) Application number: 23934690.1
(22) Date of filing: 17.05.2023
(51) Int. Cl.: G01N 27/327, G01N 21/64, C12Q 1/6825

(54) **CRISPR/CAS-BASED ELECTROCHEMICAL NUCLEIC ACID DETECTION SENSING ELEMENT AND DETECTION METHOD**

(30) Priority: 25.04.2023 CN 202310454963
(71) Applicant: Suzhou Zhong Ke Su Jing Biotechnology Co., Ltd., Suzhou, Jiangsu 215531 (CN); Institute of Process Engineering, Chinese Academy of Sciences, Beijing 100190 (CN)
(72) Inventor: ZHOU, Lei, Suzhou, Jiangsu 215531 (CN); DONG, Jinying, Suzhou, Jiangsu 215531 (CN); WU, Xiaoya, Suzhou, Jiangsu 215531 (CN); SHAO, Gaoxiang, Suzhou, Jiangsu 215531 (CN); ZHANG, Yue, Suzhou, Jiangsu 215531 (CN); MENG, Fanwei, Suzhou, Jiangsu 215531 (CN); DU, Yingxia, Suzhou, Jiangsu 215531 (CN); HU, Qiushi, Suzhou, Jiangsu 215531 (CN); SUN, Chongsi, Suzhou, Jiangsu 215531 (CN)
(74) Representative: Bryn Aarflot AS
(86) International application number: PCT/CN2023/094842
(87) International publication number: WO 2024/221503

(57) **Abstract**

The present application relates to the field of electrochemical analysis and detection, and provides a CRISPR/Cas-based electrochemical nucleic acid detection sensing element and a detection method. The element comprises non-immobilized reporter molecules, and each reporter molecule comprises at least one electrochemically active molecule and at least one nucleotide, and is a non-specific nucleic acid molecule. The present application does not need to immobilize the reporter molecules on the surface of an electrode, and can establish a homogeneous-phase, real-time and non-immobilized CRISPR/Cas sensing system, thereby realizing real-time monitoring of an electrochemical nucleic acid detection process under a homogeneous phase and repeated detection of an electrode on an electrochemical nucleic acid system under the homogeneous phase flow.

## Description

### CROSS REFERENCE TO RELATED APPLICATIONS

The present application claims the benefit of priority to the Chinese Patent Application No. 202310454963.8 filed with China National Intellectual Property Administration on April 25, 2023 and entitled "CRISPR/Cas-based Electrochemical Nucleic Acid Detection Sensing Element and Detection Method", which is incorporated herein by reference in its entirety.

### TECHNICAL FIELD

The present disclosure relates to the field of electrochemical analysis and detection, and in particular to a CRISPR/Cas-based electrochemical nucleic acid detection sensing element and a detection method.

### BACKGROUND

In recent years, polymerase chain reaction (PCR) technology has been widely used as a primary nucleic acid detection method on a large scale. However, it still suffers from several significant defects, including cumbersome procedures, low portability of instruments, limited detection sensitivity, and a strong dependence on skilled personnel and specialized environments. The development of a new generation of simpler and faster nucleic acid detection technologies holds great promise for enabling testing outside of specialized laboratories and skilled personnel.

The clustered regularly interspaced short palindromic repeats (CRISPR) and CRISPR-associated proteins (Cas) together constitute a CRISPR/Cas system, a new generation of gene-editing tools based on nucleic acid cleavage, which has attracted widespread attention since its discovery. With its significant advantages such as high sensitivity, strong specificity, rapid reaction rate, simple operation, and high efficiency, the CRISPR/Cas tool not only plays a unique role in gene editing but also provides new possibilities for nucleic acid detection. The CRISPR/Cas nucleic acid detection tool is formed by a Cas protein, CRISPR RNA (crRNA), and a reporter molecule. The Cas protein and the crRNA are capable of forming a stable binary complex. Depending on the properties of the Cas protein (such as Cas9, Cas12, Cas13, or Casl4), crRNA can selectively bind to RNA or single-stranded DNA (ssDNA) in a sample, forming a ternary complex of Cas/crRNA/RNA or Cas/crRNA/ssDNA. The ternary complex induces conformational changes in the Cas protein that confer trans-cleavage activity toward RNA or ssDNA substrates. The reporter molecule is formed by a DNA or RNA sequence conjugated with a signal molecule. The Cas ternary complex cleaves the DNA or RNA sequence within the reporter molecule, and a free signal molecule is released, thereby generating a detectable optical or colorimetric signal.

Traditional CRISPR/Cas tools have always focused on the integration of biomolecular specific recognition and optical signal response, that is, a fluorescent substance is used as the signal molecule in the reporter molecule. However, optical signal detection relies on complex optical systems, which increases instrument size and detection costs, thereby limiting its widespread application. New CRISPR/Cas tools are gradually focusing on the simplicity, speed and high sensitivity of electrochemical detection, which may bring further performance improvements to CRISPR/Cas nucleic acid detection. Electrochemical instruments are compact in size, simple in structure, low in cost, rapid in response, and high in detection sensitivity. Moreover, electrochemical instruments represented by blood glucose meters have been successfully productized and commercialized.

### SUMMARY

In view of the foregoing deficiencies, the present disclosure aims to provide a CRISPR/Cas-based electrochemical nucleic acid detection sensing element and a detection method.

Therefore, the present disclosure implements the following technical solutions:
a CRISPR/Cas-based electrochemical nucleic acid detection sensing element, including a non-immobilized reporter molecule, where the non-immobilized reporter molecule is a non-specific nucleic acid molecule, and the reporter molecule includes:
at least one electrochemically active molecule; and
at least one nucleotide.

The reporter molecule is a non-specific nucleic acid molecule, that is, the non-specific nucleic acid molecule that does not engage in pairing recognition.

Optionally, the reporter molecule includes at least one modification group;
the modification group is modified at one end, in a middle, or at both ends of the reporter molecule; and/or
the modification group is a macromolecule having steric hindrance that does not produce an interference peak near a redox peak of an electrochemically active substance.
the modification group is at least one of carboxyfluorescein, biotin, and digoxigenin; and/or
the electrochemically active molecule is modified at one end, a middle, or both ends of the reporter molecule; and/or
the electrochemically active molecule includes at least one of ferrocene, methylene blue, and thionine.

Optionally, the non-specific nucleic acid molecule is a DNA strand, an RNA strand, or a DNA/RNA hybrid strand; and/or
a length of the non-specific nucleic acid molecule is 1-26 bases.

Optionally, the CRISPR/Cas-based electrochemical nucleic acid detection sensing element further includes a Cas protein, crRNA, and/or a reaction buffer.

Optionally, the Cas protein includes at least one of Cas9, Cas12, Casl3, and Cas14; and/or
a molar concentration ratio of the Cas protein to the crRNA is 4:1-1:4; and/or
a concentration of the reporter molecule is 1-50 µM; and/or
the reaction buffer includes 10-50 mM Tris-HCl, 30-120 mM KCl or NaCl, 1-20 mM MgCl₂, and 80-130 µg/mL BSA.

Optionally, the molar concentration ratio of the Cas protein to the crRNA is 2:1; and/or
the concentration of the reporter molecule is 5-10 µM; and/or
the reaction buffer includes 20-40 mM Tris-HCl, 40-80 mM KCl or NaCl, 1.5-10 mM MgCl₂, and 90-100 µg/mL BSA.

A CRISPR/Cas-based electrochemical nucleic acid detection method, including:
constructing a CRISPR/Cas reaction system using the CRISPR/Cas-based electrochemical nucleic acid detection sensing element, adding a sample to be detected for reaction, and performing real-time electrochemical signal detection using an unmodified electrode.

Optionally, a reaction duration is 5-60 min, and a reaction temperature is 5-50°C; and/or
the unmodified electrode includes a screen-printed electrode.

Optionally, the reaction duration is 15-30 min, and the reaction temperature is 20-40°C; and/or
the unmodified electrode includes a gold electrode, a platinum electrode, a graphite electrode, or a carbon electrode.

The technical solutions of the present disclosure have the following advantages:
1. The present disclosure provides a CRISPR/Cas-based electrochemical nucleic acid detection sensing element, including a non-immobilized reporter molecule; the non-immobilized reporter molecule is a non-specific nucleic acid molecule, and the reporter molecule includes at least one electrochemically active molecule; and at least one nucleotide; and the reporter molecule is a non-specific nucleic acid molecule that does not engage in pairing recognition. During research, the present disclosure discovered that in the electrochemical nucleic acid detection based on CRISPR/Cas, one end of the reporter molecule is generally connected to an electrochemically active molecule, and the other end thereof is immobilized on the electrode surface. As the reaction proceeds, the reporter molecule is cleaved, and the electrochemically active substance is released and moves away from the electrode surface, leading to a decrease in the electrical signal and enabling electrochemical detection. The research concept has been limited to the strategy of "immobilizing on electrode and releasing away from electrode". However, the present disclosure discovered that the strategy of "immobilizing on electrode and releasing away from electrode" results in several drawbacks. Specifically, in terms of "precision and repeatability", since the reporter molecule must be immobilized on the electrode surface, leading to complex electrode fabrication, significant batch-to-batch variation, and the inability to reuse. In terms of "sensitivity and specificity", the solid-liquid reaction mode limits the molecular Brownian motion and collision efficiency, and fails to prevent nonspecific collisions from occurring, resulting in low cleavage efficiency of the reporter molecule during sensitive identification, and generating interference signals by collisions between free active molecules and electrodes during specific detection. In terms of "quantitative capability and detection limit", the inverse correlation between detection signal and target concentration makes it difficult to eliminate high noise in the detection results, resulting in poor linearity of the response, difficulty in extracting weak signals, and an unsatisfactory detection limit, which collectively impose serious constraints on the improvement of detection performance. The present disclosure discovered that when the reporter molecule is non-immobilized and freely dispersed in the reaction system, both the reporter molecule and the CRISPR/Cas complex exist in a homogeneous phase, which significantly improves molecular collision efficiency and consequently increases cleavage efficiency. Furthermore, after the reporter molecule is cleaved, the released molecules of electrochemically active substance are smaller in size and have a higher diffusion rate. This allows them to generate signals upon collision with the electrode more effectively than the reporter molecule, resulting in a clear electrical signal intensity that is positively correlated with the target concentration, thereby achieving higher sensitivity and establishing a positive correlation response. In summary, the present disclosure constructs an electrochemical nucleic acid detection sensing element based on CRISPR/Cas that features fast signal response, simple structure, and enables simple, sensitive, rapid, accurate, and cost-effective detection of target nucleic acid, and has a wide range of applications and high portability. In summary, the present disclosure replaces the conventional strategy of electrode-immobilized reporter molecules by establishing a homogeneous CRISPR/Cas recognition system without immobilization, which enables the electrochemical detection to have a low background signal and an excellent signal-to-noise ratio, thereby achieving high detection accuracy and sensitivity, and significantly improving the detection performance.
2. The present disclosure provides a CRISPR/Cas-based electrochemical nucleic acid detection sensing element, where the reporter molecule includes at least one modification group, the modification group is modified at one end, in a middle, or at both ends of the reporter molecule, and/or the modification group is a macromolecule having steric hindrance that does not produce an interference peak near a redox peak of an electrochemically active substance. During research, the present disclosure discovered that when the modification group is introduced onto the reporter molecule that is linked to the electrochemically active molecule, the steric hindrance of the reporter molecule increases. The greater steric hindrance, along with the wrapping and entanglement of the modification group around nucleic acid molecule, hinders electron transfer of the electrochemically active molecule, thereby effectively eliminating the background signal from free reporter molecules and enabling the extraction of weak signals with low detection limits. Accordingly, based on the mechanism discovered above, the reporter molecule can be freely dispersed in the CRISPR/Cas reaction system without immobilization. In addition, the reporter molecules are modified not only with electrochemically active moieties but also with additional modification groups. Before the reporter molecule is cleaved, the presence of the modification group of the reporter molecule leads to extremely weak electrical signals and reduced background signals. After the reporter molecule is cleaved, the released electrochemically active molecule is released, exhibits reduced steric hindrance and full dissociation, increased diffusion coefficient and enhanced electrode contact probability, thereby greatly enhancing the electrical signal and producing a strong specific recognition signal. In summary, the present disclosure replaces the conventional concept of using simple unmodified reporter molecules and constructs a diversely modified reporter-molecule sensing element, which reduces background noise, enhances the detection of weak signals, and further improves the sensitivity of homogeneous detection.
3. The present disclosure provides a CRISPR/Cas-based electrochemical nucleic acid detection method, including the steps of constructing a CRISPR/Cas reaction system using the CRISPR/Cas-based electrochemical nucleic acid detection sensing element, adding a sample to be detected for reaction, and performing real-time electrochemical signal detection using an unmodified electrode. The electrochemical nucleic acid detection method breaks the traditional limitations of electrode modification on the precision, reproducibility, and repeatability of electrochemical detection technologies, thereby realizing real-time monitoring of an electrochemical nucleic acid detection process under a homogeneous phase and repeated detection of an electrode on an electrochemical nucleic acid system under the homogeneous phase flow. In the application of CRISPR/Cas tools for nucleic acid detection, the coupling of a reporter molecule with a modification group and an electrochemically active molecule enables homogeneous, real-time, and repeatable detection with electrochemical analysis. The method not only improves detection performance but also provides simple, fast, and low-cost practical capabilities.

### BRIEF DESCRIPTION OF THE DRAWINGS

In order to more clearly illustrate technical solutions in the specific implementations of the present disclosure or in the prior art, a brief introduction to the accompanying drawings required for the description of the specific implementations or the prior art will be provided below. Obviously, the accompanying drawings in the following description are some of the implementations of the present disclosure, and those of ordinary skill in the art may still derive other drawings from these accompanying drawings without any creative effort.
FIG. 1 illustrates electrical signal detection results in Test Example 1 using the methods of Examples 1 and 7 according to the present disclosure.
FIG. 2 illustrates electrical signal detection results in Test Example 2 using the methods of Examples 4 and 8 according to the present disclosure.

### DETAILED DESCRIPTIONS OF THE EMBODIMENTS

The following embodiments are provided to facilitate a better understanding of the present disclosure. However, these embodiments are not intended to limit the content and scope of protection of the present disclosure. Any products identical or substantially similar to those of the present disclosure that are derived from the teachings of the present disclosure by combining features of the present disclosure with other prior art techniques by any person should fall within the scope of protection of the present disclosure.

For test steps or conditions not specifically indicated in the embodiments, the conventional test steps or conditions described in the literature of the field can be followed. Reagents or instruments used without indicating manufacturers are conventional products commercially available.

### Example 1

This example provides a CRISPR/Cas-based electrochemical nucleic acid detection sensing element, including:
a reporter molecule: is formed by an RNA strand, ferrocene (Fc) and biotin; a sequence of the RNA strand is consistent with the cleavage preference of the Cas13a protein and has a length of 6 bases, a nucleotide sequence of the reporter molecule corresponds to a nucleotide sequence (see the biotin reporter in the table under ssRNA reporter in Table S1 therein) of the biotin reporter gene in the Cas13a-SHERLOCK (Ding R, Shen Y, Yuan M, Zheng X, Chen S, Duan G. Rapid and facile detection of HBV with CRISPR/Cas 13a. New Journal of Chemistry, 2022; 46(41) : 19997-20004), with the biotin modified at a 5' end, and the ferrocene (Fc) modified at a 3' end.

A preparation method of the reporter molecule includes the following steps:
(1) a solid-phase CPG support labeled with biotin at a 5' end (commercially available) was loaded into an automated synthesizer (ABI, Model 394), and an oligonucleotide chain was synthesized in a 5'→3' direction. After completion of synthesis, the CPG powder was collected;
(2) 0.5 mL of 25% aqueous ammonia was added to the CPG support, followed by incubation at 55°C for 8 h to allow the oligonucleotide chain to be released from the solid-phase support;
(3) 2 equivalents of 1 mol/L tetrabutylammonium fluoride (TBAF) solution in tetrahydrofuran (THF) were added and incubated at room temperature for 5 h to expose a silanol group at a 2' position;
(4) after impurities were removed, the resulting product and 5 equivalents of 0.5 mol/L Fc N-hydroxysuccinimide ester (Fc-NHS) in dimethyl sulfoxide (DMSO) solution were added in a sodium bicarbonate solution (pH = 9) for incubation at 50°C for 8 h, to yield a desired sequence; and
(5) a resulting sample was subjected to ultrafiltration, followed by purification using preparative high-performance liquid chromatography (HPLC) to obtain the reporter molecule.

The constructed CRISPR/Cas13a reaction system includes:
7 µM reporter molecule, 40 nM Cas13a protein, 20 nM crRNA, 20 mM Tris-HCl (pH 8.0), 50 mM KCl, 2.5 mM MgCl₂, and 100 µg/mL BSA, with the remaining volume adjusted with ultrapure water.

This example further provides an electrochemical nucleic acid detection method using the aforesaid CRISPR/Cas-based electrochemical nucleic acid detection sensing element, including:
a gold electrode was connected to an electrochemical workstation, and immersed in a CRISPR/Cas13a reaction system. 6 µL of a sample to be detected was added to the CRISPR/Cas 13a reaction system, and incubated at 37°C for 25 min. The sample to be detected may be either an extraction-free product or an extracted and purified nucleic acid. After the reaction was completed, an electrical signal of ferrocene in the CRISPR/Cas13a reaction system was detected using a square wave voltammetry.

The detection principle of the method is as follows: When a target RNA is present in the sample to be detected, the target RNA forms a ternary complex with the Cas13a protein and crRNA, which activates the trans-cleavage activity of the Casl3a protein, cleaving an RNA strand in the reporter molecule and releasing ferrocene. In contrast, when no target RNA is present in the sample to be detected, the trans-cleavage activity of the Cas13a protein remains inactivated, and an RNA strand in the reporter molecule is not cleaved. Consequently, ferrocene modified therein cannot be released. Since a free and intact reporter molecule is modified with a group, it creates significant steric hindrance and is surrounded by nucleic acid and a modification group. The ferrocene modified therein cannot transfer electrons to an electrode. Therefore, when no target RNA is present in the sample to be detected, an electrical signal of ferrocene in the CRISPR/Cas13a reaction system is extremely weak, resulting in a low background signal. Conversely, the ferrocene itself is small in volume, diffuses rapidly, and exhibits minimal steric hindrance. Therefore, when a target RNA is present in the sample to be detected, an electrical signal of ferrocene in the CRISPR/Cas13a reaction system is relatively strong. Overall, the method has strong detection performance.

### Example 2

This example differs from Example 1 in that the constructed CRISPR/Cas13a reaction system includes:
1 µM reporter molecule, 10 nM Cas13a protein, 40 nM crRNA, 50 mM Tris-HCl, 30 mM KCL, 1 mM MgCl₂, and 80 µg/mL BSA, with the remaining volume adjusted with ultrapure water.

### Example 3

This example differs from Example 1 in that the constructed CRISPR/Cas13a reaction system includes:
5 µM reporter molecule, 40 nM Cas13a protein, 10 nM crRNA, 40 mM Tris-HCl, 40 mM KCL, 1.5 mM MgCl₂, and 90 µg/mL BSA, with the remaining volume adjusted with ultrapure water.

### Example 4

This example provides a CRISPR/Cas-based electrochemical nucleic acid detection sensing element, including:
a reporter molecule: is formed by a DNA strand, carboxyfluorescein (FAM) and ferrocene (Fc); a sequence of the DNA strand is consistent with the cleavage preference of a CRISPR/Cas 12a protein and has a length of 10 bases. A nucleotide sequence of the reporter molecule corresponds to a nucleotide sequence (see the surface probe-10nt in the table of the materials therein) of a surface probe in E-CRISPR (Dai Y, Somoza RA, Wang L, Welter JF, Li Y, Caplan AI, et al. Exploring the Trans-Cleavage Activity of CRISPR-Cas12a (cpfl) for the Development of a Universal Electrochemical Biosensor. Angew Chem Int Ed Engl. 2019; 58(48):17399-405), with the FAM modified at a 5' end, and the Fc modified at a 3' end.

A preparation method of the reporter molecule includes the following steps:
(1) a solid-phase CPG support labeled with Fc at the 3' end was loaded into an automated synthesizer (ABI, Model 394), an oligonucleotide chain was synthesized in a 3'→5' direction, and a desired sequence was obtained by coupling with a commercially available FAM phosphoramidite reagent for incubating at 50°C for 8 h;
(2) a resulting product was released and deprotected using a mixture of tert-butylamine : methanol : water (1:1:2, v/v/v), followed by incubation at 65°C in a water bath for 3 h; and
(3) a resulting sample was subjected to ultrafiltration, followed by purification using preparative HPLC to obtain the reporter molecule.

The constructed CRISPR/Cas12a reaction system includes:
7 µM reporter molecule, 40 nM Cas12a protein, 20 nM crRNA, 30 mM Tris-HCl (pH 7.9), 70 mM NaCl, 10 mM MgCl₂, and 100 µg/mL BSA, with the remaining volume adjusted with ultrapure water.

This example further provides an electrochemical nucleic acid detection method using the aforesaid CRISPR/Cas-based electrochemical nucleic acid detection sensing element, including:
a gold electrode was connected to an electrochemical workstation, and immersed in a CRISPR/Cas12a reaction system. 6 µL of a sample to be detected was added to the CRISPR/Cas 12a reaction system, and incubated at 37°C for 25min. The sample to be detected may be either an extraction-free product or an extracted and purified nucleic acid. After the reaction was completed, an electrical signal of ferrocene in the CRISPR/Cas12a reaction system was detected using a differential pulse voltammetry.

### Example 5

This example differs from Example 4 in that the constructed CRISPR/Cas12a reaction system includes:
50 µM reporter molecule, 40 nM Cas12a protein, 10 nM crRNA, 10 mM Tris-HCl, 120 mM NaCl, 10 mM MgCl₂, and 100 µg/mL BSA, with the remaining volume adjusted with ultrapure water.

### Example 6

This example differs from Example 4 in that the constructed CRISPR/Cas12a reaction system includes:
10 µM reporter molecule, 10 nM Cas12a protein, 40 nM crRNA, 20 mM Tris-HCl, 80 mM NaCl, 20 mM MgCl₂, and 130 µg/mL BSA, with the remaining volume adjusted with ultrapure water.

### Example 7

This example differs from Example 1 in that the reporter molecule does not contain biotin as a modification group.

In the step (1) of the reporter molecule preparation method, a common solid-phase CPG support (that is, a solid-phase CPG support not labeled with biotin at a 5' end) was used.

### Example 8

This example differs from Example 4 in that the reporter molecule does not contain carboxyfluorescein (FAM) as a modification group.

In the step (1) of the reporter molecule preparation method, FAM was not added as a synthetic raw material.

### Test Example 1

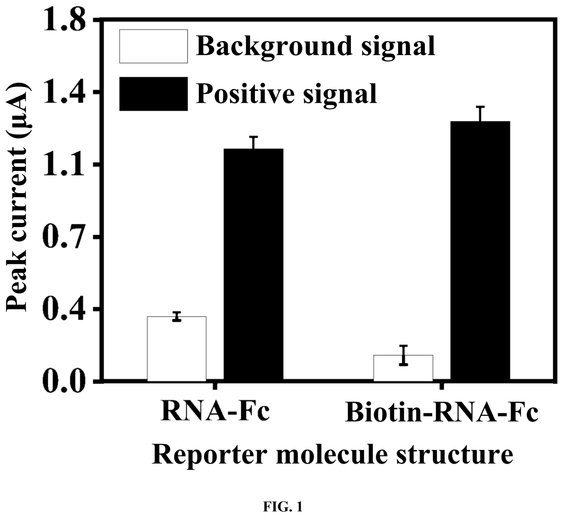

Target sequence and crRNA sequence were ssRNA1 and crRNA1 shown in FIG. 1 of SHERLOCK (see Gootenberg JS, Abudayyeh OO, Lee JW, Essletzbichler P, Dy AJ, Joung J, et al. Nucleic acid detection with CRISPR-Cas13a/C2c2. Science. 2017; 356(6336):438-42.). Subsequent operation was implemented according to Example 1 and Example 7, as described below:
(1) A CRISPR/Cas13a reaction system was constructed as follows:
   7 µM reporter molecule, 40 nM Cas13a protein, 20 nM crRNA, 20 mM Tris-HCl (pH 8.0), 50 mM KCl, 2.5 mM MgCl₂, and 100 µg/mL BSA, with the remaining volume adjusted with ultrapure water.
(2) A gold electrode was connected to an electrochemical workstation, and immersed in a CRISPR/Cas13a reaction system.
(3) 6 µL of extracted and purified nucleic acid (with a concentration approximately 10 copies/µL) was added to the constructed CRISPR/Cas13a reaction system, and incubated at 37°C for 25 min.
(4) An electrical signal of ferrocene in the CRISPR/Cas13a reaction system was detected using a square wave voltammetry.

Results are shown in FIG. 1. The biotin-RNA-Fc in the figure is the reporter molecule in Example 1, and the RNA-Fc is the reporter molecule in Example 7. As can be seen from the figure, the reporter molecules in Example 1 and Example 7 both produced strong positive signals and weak background signals. Further comparison between Example 1 and Example 7 revealed that Example 1 exhibited a lower background signal and a higher positive signal, indicating that the biotin-RNA-Fc reporter molecule in Example 1 provided a lower background signal and a better signal-to-noise ratio.

### Test Example 2

Target sequence and crRNA sequence were LbCas 12a-crRNA-HPV 16 and HPV-16 in E-CRISPR (see Dai Y, Somoza RA. Wang L, Welter JF, Li Y, Caplan AI, et al. Exploring the Trans-Cleavage Activity of CRISPR-Cas12a (cpfl) for the Development of a Universal Electrochemical Biosensor. Angew Chem Int Ed Engl. 2019; 58(48): 17399-405.). Subsequent operation was implemented according to Example 4 and Example 8, as described below:
(1) A CRISPR/Cas12a reaction system was constructed as follows:
   7 µM reporter molecule, 40 nM Cas12a protein, 20 nM crRNA, 30 mM Tris-HCl (pH 7.9), 70 mM NaCl, 10 mM MgCl₂, and 100 µg/mL BSA, with the remaining volume adjusted with ultrapure water.
(2) A gold electrode was connected to an electrochemical workstation, and immersed in a CRISPR/Cas12a reaction system.
(3) 6 µL of extracted and purified nucleic acid (with a concentration approximately 10 copies/µL) was added to the constructed CRISPR/Cas12a reaction system, and incubated at 37°C for 25 min.
(4) An electrical signal of ferrocene in the CRISPR/Cas12a reaction system was detected using a differential pulse voltammetry.

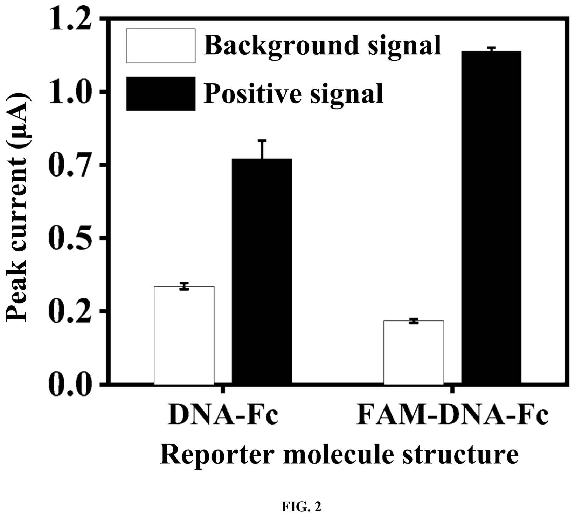

Results are shown in FIG. 2. The FAM-DNA-Fc in the figure is the reporter molecule in Example 4, and the DNA-Fc is the reporter molecule in Example 8. As can be seen from the figure, the reporter molecules in Examples 4 and 8 both produced strong positive signals and weak background signals. Further comparison between Example 4 and Example 8 revealed that Example 4 exhibited a lower background signal and a higher positive signal, indicating that the FAM-DNA-Fc reporter molecule in Example 4 provided a lower background signal and a better signal-to-noise ratio.

Apparently, the above embodiments are merely examples given for clearly illustrating the present invention, and are not intended to limit the implementations. For those of ordinary skill in the art, modifications or variations in other forms may be made on the basis of the above description. It is unnecessary to exhaust all implementations herein. Obvious modifications or variations made thereto shall still fall within the scope of protection of the present invention.

## Claims

1. A CRISPR/Cas-based electrochemical nucleic acid detection sensing element, comprising a non-immobilized reporter molecule, wherein the non-immobilized reporter molecule is a non-specific nucleic acid molecule, and the reporter molecule comprises:
at least one electrochemically active molecule; and
at least one nucleotide.

2. The CRISPR/Cas-based electrochemical nucleic acid detection sensing element according to claim 1, wherein the reporter molecule contains at least one modification group:
the modification group is modified at one end, in a middle, or at both ends of the reporter molecule; and/or
the modification group is a macromolecule having steric hindrance that does not produce an interference peak near a redox peak of an electrochemically active substance.

3. The CRISPR/Cas-based electrochemical nucleic acid detection sensing element according to claim 2, wherein
the modification group is at least one of carboxyfluorescein, biotin, and digoxigenin; and/or
the electrochemically active molecule is modified at one end, a middle, or both ends of the reporter molecule; and/or
the electrochemically active molecule comprises at least one of ferrocene, methylene blue, and thionine.

4. The CRISPR/Cas-based electrochemical nucleic acid detection sensing element according to claim 1 or 2, wherein the non-specific nucleic acid molecule is a DNA strand, an RNA strand, or a DNA/RNA hybrid strand; and/or
a length of the non-specific nucleic acid molecule is 1-26 bases.

5. The CRISPR/Cas-based electrochemical nucleic acid detection sensing element according to claim 1 or 2, further comprising a Cas protein, crRNA, and/or a reaction buffer.

6. The CRISPR/Cas-based electrochemical nucleic acid detection sensing element according to claim 5, wherein the Cas protein comprises at least one of Cas9, Cas12, Cas13, and Cas14; and/or
a molar concentration ratio of the Cas protein to the crRNA is 4:1-1:4; and/or
a concentration of the reporter molecule is 1-50 µM; and/or
the reaction buffer comprises 10-50 mM Tris-HCl, 30-120 mM KCl or NaCl, 1-20 mM MgCl₂, and 80-130 µg/mL BSA.

7. The CRISPR/Cas-based electrochemical nucleic acid detection sensing element according to claim 6, wherein the molar concentration ratio of the Cas protein to the crRNA is 2:1; and/or
the concentration of the reporter molecule is 5-10 µM; and/or
the reaction buffer comprises 20-40 mM Tris-HCl, 40-80 mM KCl or NaCl, 1.5-10 mM MgCl₂, and 90-100 µg/mL BSA.

8. A CRISPR/Cas-based electrochemical nucleic acid detection method, comprising:
constructing a CRISPR/Cas reaction system using the CRISPR/Cas-based electrochemical nucleic acid detection sensing element according to any one of claims 1-7, adding a sample to be detected for reaction, and performing real-time electrochemical signal detection using an unmodified electrode.

9. The CRISPR/Cas-based electrochemical nucleic acid detection method according to claim 8, wherein a reaction duration is 5-60 min, and a reaction temperature is 5-50°C; and/or
the unmodified electrode comprises a screen-printed electrode.

10. The CRISPR/Cas-based electrochemical nucleic acid detection method according to claim 9, wherein the reaction duration is 15-30 min, and the reaction temperature is 20-40°C; and/or
the unmodified electrode comprises a gold electrode, a platinum electrode, a graphite electrode, or a carbon electrode.
